# EUROPÄISCHE PATENTSCHRIFT

(11) **EP 1 948 257 B1**
(45) Veröffentlichungstag und Bekanntmachung des Hinweises auf die Patenterteilung: **22.07.2009**
(21) Anmeldenummer: 06818566.9
(22) Anmeldetag: 16.11.2006
(51) Int. Cl.: A61L 27/22

(54) **FORMKÖRPER AUF BASIS EINES VERNETZTEN, GELATINE ENTHALTENDEN MATERIALS, VERFAHREN ZU DEREN HERSTELLUNG SOWIE DEREN VERWENDUNG**
SHAPED BODIES BASED ON A CROSS-LINKED, GELATINOUS MATERIAL, METHOD FOR PRODUCING THE SAME AND THEIR USE
CORPS MOULE A FABRIQUE A PARTIR D'UN MATERIAU RETICULE, CONTENANT DE LA GELATINE, PROCEDE DE PRODUCTION ET D'UTILISATION ASSOCIES

(30) Priorität: 17.11.2005 DE 102005054938
(43) Veröffentlichungstag der Anmeldung: 30.07.2008
(73) Patentinhaber: GELITA AG, 69412 Eberbach (DE)
(72) Erfinder: AHLERS, Michael, 69412 Eberbach (DE); RUPP, Melanie, 69412 Eberbach (DE)
(74) Vertreter: Wössner, Gottfried
(86) Internationale Anmeldenummer: PCT/EP2006/010973
(87) Internationale Veröffentlichungsnummer: WO 2007/057176

(56) Entgegenhaltungen:
- WO-A-93/13753
- WO-A-98/19718
- US-B1- 6 608 040
- A. BIGI ET AL: "Drawn gelatin films with improved mechanical proprties" BIOMATERIALS, Bd. 19, Nr. 24, Dezember 1998 (1998-12), Seiten 2335-2340, XP002426606

## Beschreibung

Die vorliegende Erfindung betrifft Formkörper auf Basis eines vernetzten, Gelatine enthaltenden Materials. Ferner betrifft die Erfindung ein Verfahren zur Herstellung derartiger Formkörper.

Die Erfindung betrifft darüber hinaus die Verwendung dieser Formkörper im medizinischen Bereich, insbesondere zur Herstellung von Implantaten.

In verschiedenen Bereichen der Medizin kommen Formkörpern aus resorbierbaren Materialien zum Einsatz, einerseits zur Abdeckung von Wunden oder von inneren oder äußeren Blutungen, sowie auch zur Herstellung von Implantaten, die eine Träger-, Stütz- oder Leitfunktion erfüllen. Besondere Bedeutung kommt dabei so genannten Gewebeimplantaten zu, bei denen es sich um Konstrukte aus einem resorbierbaren Trägermaterial und lebenden Zellen handelt (Tissue Engineering). Diese dienen zur Behandlung von geschädigten Geweben und Organen, insbesondere zur Regeneration von Haut oder Knorpel.

Derartige Materialien müssen eine Reihe von Eigenschaften erfüllen, um im medizinischen Bereich erfolgreich eingesetzt werden zu können. Einerseits müssen sie eine ausreichende Festigkeit aufweisen, um eine beschädigungsfreie Handhabung zu ermöglichen, und um anwachsende Zellen im Körper vor mechanischer Beanspruchung zu schützen. Gleichzeitig sollte das Material aber flexibel genug sein, um sich der Form der zu behandelnden Körperstelle anzupassen.

Es wurde gefunden, dass zur Erfüllung der genannten Voraussetzungen Gelatine als Basismaterial gut geeignet ist. Gelatine kann vom Körper vollständig resorbiert werden und weist damit einen Vorteil gegenüber anderen Materialien wie beispielsweise Chitosan, Alginat, Agarose und Hyaluronsäure auf. Im Gegensatz zu dem verwandten Material Kollagen ist Gelatine in hoher Reinheit und mit reproduzierbarer Zusammensetzung erhältlich und ist frei von immunogenen Telopeptiden, die Abwehrreaktionen des Körpers auslösen können.

Um eine ausreichend lange Stabilität der Formkörper unter physiologischen Bedingungen zu erreichen, muss die Gelatine in der Regel chemisch oder enzymatisch vernetzt werden. Die rückstandsfreie Resorbierbarkeit wird dadurch nicht beeinträchtigt, die jeweilige Resorptionszeit kann aber durch den Grad der Vernetzung individuell eingestellt werden.

Ein Verfahren zur Herstellung derartiger Formkörper auf Basis von vernetzter Gelatine ist in der deutschen Patentanmeldung mit dem Aktenzeichen DE 10 2004 024 635 beschrieben.

Für bestimmte Anwendungen ist jedoch eine sehr hohe Festigkeit der Formkörper wünschenswert, die allein durch eine Erhöhung des Vernetzungsgrades nicht erreicht werden kann.

Es wurde beschrieben, dass die Reißfestigkeit von Gelatinefolien durch eine Verstreckung der Folien erhöht werden kann (Bigi et al. (1998) Biomaterials 19, 2335-2340). Allerdings wiesen die in dieser Veröffentlichung beschriebenen Folien, die nach dem Verstrecken mit Glutaraldehyd vernetzt wurden, eine Reißdehnung unterhalb von 11% auf. Derartige Folien verfügen nicht über die für eine medizinische Anwendung wünschenswerte Flexibilität.

Die Aufgabe der vorliegenden Erfindung besteht darin, Formkörper auf Basis von Gelatine zur Verfügung zu stellen, die sowohl eine hohe mechanische Festigkeit als auch eine ausreichende Flexibilität aufweisen.

Diese Aufgabe wird erfindungsgemäß gelöst durch einen Formkörper auf Basis eines vernetzten, Gelatine enthaltenden Materials, wobei der Formkörper verstreckt ist, so dass die Gelatinemoleküle zumindest teilweise in einer Vorzugrichtung orientiert sind, und wobei das Material einen Weichmacher umfasst.

Überraschenderweise können Formkörper auf Basis von Gelatine, die einerseits einen Weichmacher umfassen und andererseits vernetzt sind, besonders gut verstreckt werden. Durch die Verstreckung können die mechanischen Eigenschaften der Formkörper, insbesondere deren Reißfestigkeit und Reißdehnung, merklich verbessert werden.

Das Gelatine enthaltende Material, auf dessen Basis der Formkörper hergestellt ist, ist bevorzugt zu einem überwiegenden Teil aus Gelatine gebildet. Darunter fallen insbesondere Gelatineanteile von 60 Gew.% oder mehr, bevorzugt von 75 Gew.% oder mehr. Neben Gelatine kann das Material beispielsweise noch weitere Biopolymere wie etwa Alginate oder Hyaluronsäure enthalten, um das Eigenschaftsprofil der Formkörper noch spezifischer an eine bestimmte Applikation anzupassen.

Um eine optimale Bioverträglichkeit der erfindungsgemäßen Formkörper bei der medizinischen Anwendung zu gewährleisten-, wird als Ausgangsmaterial bevorzugt eine Gelatine mit einem besonders geringen Gehalt an Endotoxinen eingesetzt. Bei Endotoxinen handelt sich um Stoffwechselprodukte oder Bruchstücke von Mikroorganismen, welche in dem tierischen Rohmaterial vorkommen. Der Endotoxingehalt von Gelatine wird in internationalen Einheiten pro Gramm (I.E./g) angegeben und gemäß dem LAL-Test bestimmt, dessen Durchführung in der vierten Ausgabe des Europäischen Arzneibuches (Ph. Eur. 4) beschrieben ist.

Um den Gehalt an Endotoxinen möglichst gering zu halten, ist es vorteilhaft, die Mikroorganismen möglichst frühzeitig im Zuge der Gelatineherstellung abzutöten. Ferner sollten entsprechende Hygienestandards beim Herstellungsprozess eingehalten werden.

Somit kann der Endotoxingehalt von Gelatine durch bestimmte Maßnahmen beim Herstellungsprozess drastisch gesenkt werden. Zu diesen Maßnahmen zählen in erster Linie die Verwendung frischer Rohmaterialien (z.B. Schweineschwarte) unter Vermeidung von Lagerzeiten, die sorgfältige Reinigung der gesamten Produktionsanlage unmittelbar vor Beginn der Gelatineherstellung sowie gegebenenfalls das Auswechseln von Ionenaustauschern und Filtersystemen in der Produktionsanlage.

Die im Rahmen der vorliegenden Erfindung eingesetzte Gelatine weist bevorzugt einen Endotoxingehalt von 1.200 I.E./g oder weniger, noch mehr bevorzugt von 200 I.E/g oder weniger auf. Optimalerweise liegt der Endotoxingehalt bei 50 I.E./g oder weniger, jeweils gemäß dem LAL-Test bestimmt. Im Vergleich hierzu weisen manche handelsübliche Gelatinen Endotoxingehalte von über 20.000 I.E./g auf.

Erfindungsgemäß umfasst das Material neben der Gelatine zumindest einen Weichmacher, wodurch die Flexibilität des Formkörpers erhöht und dessen Verstreckbarkeit deutlich verbessert wird. Als Weichmacher sind z.B. Glycerin, Oligoglycerine, Oligoglykole und Sorbit geeignet, wobei Glycerin am meisten bevorzugt ist.

Die gewünschte Flexibilität des Formkörpers kann über die Menge des Weichmachers gesteuert werden. Bevorzugt liegt der Anteil an Weichmacher in dem Material bei 12 bis 40 Gew.%. Besonders vorteilhaft sind dabei Anteile von 16 bis 25 Gew.%.

Der verstreckte Formkörper ist bevorzugt monoaxial verstreckt. Dadurch wird eine Vorzugsrichtung definiert, entlang derer die Gelatinemoleküle zumindest teilweise orientiert sind.

Die erfindungsgemäßen Formkörper weisen eine hohe mechanische Festigkeit, insbesondere Reißfestigkeit, auf. Bevorzugt haben die erfindungsgemäßen Formkörper eine Reißfestigkeit von 40 N/mm² oder mehr, weiter bevorzugt von 60 N/mm² oder mehr, jeweils gemessen in Verstreckrichtung.

Darüber hinaus weisen die Formkörper überraschenderweise auch eine hohe Reißdehnung (Dehngrenze) auf, insbesondere in Verstreckrichtung. Bevorzugt liegt dabei die Reißdehnung des Formkörpers bei 30% oder höher, weiter bevorzugt bei 50% oder höher, jeweils gemessen in Verstreckrichtung.

Grundsätzlich können sowohl die Gelatine als auch andere geeignete Komponenten des Materials in dem Formkörper vernetzt sein. Es ist jedoch bevorzugt, dass insbesondere die Gelatine vernetzt ist.

Bei der Vernetzung kann es sich um eine chemische Vernetzung handeln. Hierfür ist prinzipiell jedes Vernetzungsmittel geeignet, das eine Verknüpfung der einzelnen Gelatinemoleküle miteinander bewirkt. Bevorzugte Vernetzungsmittel sind Aldehyde, Dialdehyde, Isocyanate, Diisocyanate, Carbodiimide und Alkyldihalogenide. Besonders bevorzugt ist Formaldehyd, welches gleichzeitig eine Sterilisierung des Formkörpers bewirkt.

Bei einer weiteren Ausführungsform des erfindungsgemäßen Formkörpers ist das Material enzymatisch vernetzt. Als Vernetzungsmittel wird dabei bevorzugt das Enzym Transglutaminase eingesetzt, welches eine Verknüpfung der Glutamin- und Lysinseitenketten von Proteinen, insbesondere auch von Gelatine, bewirkt.

Die erfindungsgemäßen Formkörper können zum Teil erstaunlich lange Lebensdauern unter physiologischen Bedingungen aufweisen, wobei diese durch den Grad der Vernetzung sehr gezielt eingestellt werden können. So können erfindungsgemäße Formkörper unter physiologischen Standardbedingungen beispielsweise länger als eine Woche, länger als zwei Wochen oder länger als vier Wochen stabil bleiben.

Der Begriff der Stabilität ist hier dahingehend zu verstehen, dass der Formkörper seine ursprüngliche Form sowohl bei Lagerung in trockenem Zustand als auch während der angegebenen Zeitdauer bei physiologischen Standardbedingungen im Wesentlichen erhält und erst anschließend strukturell in erheblichem Umfang hydrolytisch abgebaut wird.

Physiologische Bedingungen, denen die Formkörper bei einer Verwendung zur Herstellung von Implantaten ausgesetzt sind, sind in erster Linie durch Temperatur, pH-Wert und Ionenstärke gekennzeichnet. Entsprechende Bedingungen können *in vitro* durch eine Inkubation in PBS-Puffer (pH 7,2, 37 °C) simuliert werden, um verschiedene Formkörper im Hinblick auf ihr zeitabhängiges Stabilitätsverhalten zu testen und zu vergleichen (im Folgenden physiologische Standardbedingungen genannt).

Die mechanische Festigkeit der erfindungsgemäßen Formkörper kann durch die Zugabe eines Verstärkungsstoffes zusätzlich erhöht werden. Die Verstärkungsstoffe sollen physiologisch verträglich und am besten ebenfalls resorbierbar sein.

Je nach Wahl des Verstärkungsstoffes lässt sich neben der Beeinflussung der mechanischen Eigenschaften auch die Stabilität der Formkörper gegen Resorptionsmechanismen in gewissem Umfang beeinflussen. Insbesondere lässt sich die Resorptionsstabilität der Verstärkungsstoffe unabhängig von den Komponenten des Gelatine enthaltenden Materials wählen.

Die Verstärkungsstoffe zeigen schon bei Anteilen von 5 Gew.% (bezogen auf die Gesamtmasse des Formkörpers) eine merkliche Verbesserung der mechanischen Eigenschaften der Formkörper.

Oberhalb von 60 Gew.% lässt sich in der Regel keine signifikante Verbesserung mehr erreichen und/oder die gewünschten Resorptionseigenschaften oder auch die notwendige Flexibilität des Formkörpers lassen sich nur noch schwierig darstellen.

Die Verstärkungsstoffe lassen sich aus partikulären und molekularen Verstärkungsstoffen sowie Mischungen hiervon auswählen.

Bei den partikulären Verstärkungsstoffen empfiehlt sich insbesondere die Verwendung von Verstärkungsfasern. Die Fasern sind dabei bevorzugt ausgewählt aus Polysaccharid- und Proteinfasem, insbesondere Kollagenfasern, Seide und Baumwollfasern, sowie aus Polylactidfasern und Mischungen hiervon.

Andererseits sind molekulare Verstärkungsstoffe ebenso geeignet, um die mechanischen Eigenschaften und, falls gewünscht, auch die Resorptionsstabilität des Formkörpers zu verbessern.

Bevorzugte molekulare Verstärkungsstoffe sind insbesondere Polylactidpolymere und deren Derivate, Cellulosederivate und Chitosan und dessen Derivate. Auch lassen sich die molekularen Verstärkungsstoffe als Mischungen einsetzen.

Bei einer bevorzugten Ausführungsform des erfindungsgemäßen Formkörpers liegt dieser als Folie vor. Derartige Folien auf Basis eines vernetzten, Gelatine enthaltenden Materials können vielfältig zum Abdecken und/oder Stützen von beschädigtem Gewebe, zur Besiedlung mit Zellen sowie zur Herstellung von Kombinationsfnaterialien in Verbindung mit Formkörpern mit einer Zellstruktur, z.B. Schwämmen, eingesetzt werden.

Die Dicke der erfindungsgemäßen Folien beträgt bevorzugt 20 bis 500 µm, am meisten bevorzugt 50 bis 250 µm.

Eine weitere bevorzugte Ausführungsform des erfindungsgemäßen Formkörpers betrifft einen Hohlzylinder. Derartige Hohlzylinder können unter anderem als Nervenleitschienen zum Einsatz kommen. Dabei handelt es sich um Implantate, die die Regeneration durchtrennter Nervenstränge erlauben, indem jeweils eine einzelne Nervenzelle entlang des Hohlraums der Nervenleitschiene wächst.

Erfindungsgemäße Hohlzylinder können sowohl in Längsrichtung als auch in Umfangsrichtung verstreckt sein. Auf die jeweilige Herstellung solcher Hohlzylinder wird weiter unten im Detail eingegangen.

Im Fall von Hohlzylindern, die in Längsrichtung verstreckt sind, werden durch das Verstrecken nicht nur deren mechanische Eigenschaften verbessert, sondern es werden gleichzeitig Hohlzylinder mit einem im Vergleich zum unverstreckten Hohlzylinder kleineren Innendurchmesser zur Verfügung gestellt. Damit kann der Innendurchmesser an die jeweiligen Anforderungen angepasst werden, z.B. an die Dimensionen von Nervenzellen bei einer Verwendung des Hohlzylindern als Nervenleitschiene.

Je nach Anwendung kann der Hohlzylinder einen Innendurchmesser von 300 bis 1.500 µm aufweisen, vorzugsweise von 900 bis 1.200 µm. Die mittlere Wandstärke des Hohlzylinders liegt vorzugsweise im Bereich von 140 bis 250 µm.

Die Aufgabe der vorliegenden Erfindung besteht ferner darin, ein Verfahren zur Verfügung zu stellen, mit dem Formkörper auf Basis von Gelatine mit verbesserten mechanischen Eigenschaften hergestellt werden können.

Diese Aufgabe wird erfindungsgemäß gelöst durch ein Verfahren, dass die folgenden Schritte umfasst:
a) Herstellen einer wässrigen Lösung eines Gelatine enthaltenden Materials;
b) partielles Vernetzen des gelösten, Gelatine enthaltenden Materials;
c) Herstellen eines Formkörpers ausgehend von der das partiell vernetzte Material enthaltenden Lösung; und
d) Verstrecken des Formkörpers.

Wie bereits im Zusammenhang mit den erfindungsgemäßen Formkörpern ausgeführt, kann durch das Verstrecken deren mechanische Festigkeit deutlich erhöht werden. Erfindungsgemäß erfolgt dabei das Verstrecken, nachdem das Gelatine enthaltende Material zumindest partiell vernetzt wurde. Diese Reihenfolge führt zu besseren Ergebnissen als ein Verstrecken des Formkörpers vor dem Vernetzen gemäß dem Stand der Technik (Bigi et al. (1998) Biomaterials 19, 2335-2340; siehe oben).

Das in Schritt a) verwendete Gelatine enthaltende Material ist bevorzugt zu einem überwiegenden Anteil aus Gelatine gebildet. Darunter fallen insbesondere Gelatineanteile von 60 Gew.% oder mehr, bevorzugt von 75 Gew.% oder mehr. Daneben kann das Material, wie oben beschrieben, noch weitere Komponenten enthalten.

Als Ausgangsmaterial für das Verfahren kann grundsätzlich Gelatine verschiedener Herkunft und Qualität eingesetzt werden; im Hinblick auf eine medizinische Anwendung ist jedoch der Einsatz von endotoxinarmer Gelatine, wie oben beschrieben, bevorzugt. Die Gelatinekonzentration in der Lösung in Schritt a) kann dabei 5 bis 45 Gew.%, vorzugsweise 10 bis 30 Gew.%, betragen.

Bevorzugt umfasst das Material in Schritt a) zusätzlich einen Weichmacher. Hierdurch wird die Verstreckbarkeit des Formkörpers wesentlich verbessert, wie dies bereits im Zusammenhang mit den erfindungsgemäßen Formkörpern beschrieben wurde.

Geeignete Weichmacher sind beispielsweise Glycerin, Oligoglycerine, Oligoglykole und Sorbit, wobei Glycerin am meisten bevorzugt ist. Vorteilhafterweise beträgt der Anteil an Weichmacher in dem Material 12 bis 40 Gew.%. Am meisten bevorzugt sind dabei Anteile von 16 bis 25 Gew.%.

Der in Schritt c) gebildete Formkörper wird vor dem Verstrecken (Schritt d)) bevorzugt zumindest teilweise getrocknet, vorzugsweise bis zu einem Restfeuchtegehalt von weniger als 20 Gew.%, insbesondere 15 Gew.% oder weniger.

Bevorzugt wird der Formkörper unmittelbar vor dem Verstrecken (Schritt d)) durch eine Erhöhung der Temperatur und/oder des Wassergehalts in einen thermoplastischen Zustand überführt. Dies kann beispielsweise dadurch erfolgen, dass der Formkörper heißem Wasserdampf ausgesetzt wird. Die Verstreckung der Formkörper wird vorteilhafterweise mit einem Verstreckungsverhältnis von 1,4 bis 8 durchgeführt, wobei ein Verstreckungsverhältnis von bis zu 4 bevorzugt ist.

Bei einer besonderen Ausführungsform des erfindungsgemäßen Verfahrens wird Schritt d) bis zu vier Wochen nach Schritt c) durchgeführt. Durch die Lagerung des Formkörpers vor dem Verstrecken, wobei bevorzugt bei Raumtemperatur gelagert wird, kann die Festigkeit der erfindungsgemäß hergestellten Formkörper zum Teil deutlich erhöht werden. Bevorzugt wird dabei Schritt d) drei bis sieben Tage nach Schritt c) durchgeführt.

Eine weitere Ausführungsform des erfindungsgemäßen Verfahrens umfasst einen weiteren Schritt e), in dem das in dem verstreckten Formkörper enthaltene Material zusätzlich vernetzt wird.

Sowohl in Schritt b) als auch in dem optionalen Schritt e) kann die Gelatine und/oder eine andere geeignete Komponente des Materials vernetzt werden. Bevorzugt wird in beiden Fällen insbesondere die Gelatine vernetzt.

Der Vorteil einer zweistufigen Vernetzung besteht grundsätzlich darin, dass ein hoher Vernetzungsgrad und damit einhergehend lange Abbauzeiten erzielt werden können. Dies kann mit einem einstufigen Verfahren unter Erhöhung der Konzentration an Vernetzungsmittel nicht in gleichem Maße verwirklicht werden, da durch ein zu starkes Vernetzen des gelösten Materials dieses nicht mehr verarbeitet und in Form gebracht werden kann.

Andererseits ist auch ein Vernetzen des Materials ausschließlich nach der Herstellung des Formkörpers nicht geeignet, da dieses hierbei an den von außen zugänglichen Grenzflächen stärker vernetzt wird als in den inneren Bereichen des Formkörpers, was sich in einem inhomogenen Abbauverhalten widerspiegelt.

Das erfindungsgemäße Verstrecken des Formkörpers zwischen den beiden Vernetzungsschritten ist besonders vorteilhaft, weil dabei die Moleküle in dem partiell vernetzten Material noch über eine ausreichende Bewegungsfreiheit verfügen und sich so zumindest teilweise entlang einer Vorzugsrichtung orientieren können.

Die zweite Vernetzung (Schritt e)) kann durch Einwirken einer wässrigen Lösung eines Vernetzungsmittels durchgeführt werden, bevorzugt ist jedoch das Einwirken eines gasförmigen Vernetzungsmittels.

In den Schritten b) und ggf. e) können gleiche oder verschiedene Vernetzungsmittel eingesetzt werden, wobei bevorzugte chemische oder enzymatische Vernetzungsmittel bereits im Zusammenhang mit dem erfindungsgemäßen Formkörper beschrieben wurden. Besonders bevorzugt ist Formaldehyd, insbesondere für den eventuellen zweiten Vernetzungsschritt in der Gasphase, da der Formkörper durch Formaldehyd gleichzeitig sterilisiert werden kann. Dabei kann das Einwirken des Formaldehyds auf den Formkörper von einer Wasserdampfatmosphäre unterstützt erfolgen.

Bevorzugt wird das Vernetzungsmittel in Schritt b) in einer Menge von 600 bis 5.000 ppm, vorzugsweise 2.000 bis 4.000 ppm, bezogen auf die Gelatine, in die Lösung zugegeben.

Durch Variation der Konzentration an Vernetzungsmittel in der Lösung, aber auch durch unterschiedlich hohe Vernetzungsgrade im zweiten Vernetzungsschritt, können sowohl die mechanische Festigkeit der hergestellten Formkörper als auch deren Lebensdauer unter physiologischen Bedingungen auf einfache Weise eingestellt werden. So können überraschenderweise Formkörper erhalten werden, die einerseits unter physiologischen Bedingungen z.B. länger als eine Woche, länger als zwei Wochen oder länger als vier Wochen stabil bleiben, und die andererseits den Anforderungen bezüglich Zellverträglichkeit und Resorbierbarkeit genügen.

Bei einer besonderen Ausführungsform des erfindungsgemäßen Verfahrens ist der Formkörper eine Folie. Folien können insbesondere durch ein Gießen oder ein Extrudieren der Lösung in Schritt c) hergestellt werden.

Bei einer anderen Ausführungsform des erfindungsgemäßen Verfahrens ist der Formkörper ein Hohlzylinder. Auch Hohlzylinder können durch ein Extrudieren der Lösung in Schritt c) hergestellt werden. Bevorzugt ist jedoch eine Herstellung von Hohlzylindern durch ein gleichmäßiges Aufbringen der Lösung in Schritt c) auf die Oberfläche eines Zylinders, insbesondere durch kurzzeitiges Eintauchen des Zylinders in die Lösung. Beim Trocknen der Lösung entsteht ein Hohlzylinder, die von dem Zylinder abgezogen werden kann.

Ein weiteres bevorzugtes Herstellungsverfahren für Hohlzylinder umfasst das Aufrollen einer Folie zu einem ein- oder mehrlagigen Hohlzylinder. Die Verbindung der Folie zu einem geschlossenen Hohlzylinder kann z.B. dadurch erfolgen, dass die Folie beim Aufrollen feucht ist und dadurch verklebt. Alternativ kann die Folie mittels eines Klebers, z.B. Gelatine, verbunden werden.

Bei einer Ausführungsform des Verfahrens wird zunächst der Hohlzylinder durch Aufrollen einer unverstreckten Folie gebildet (Schritte a) bis c)) und im Anschluss in Längsrichtung verstreckt (Schritt d)), wobei der Innendurchmesser verkleinert wird (siehe oben). Auf diese Weise können auch durch Tauchen hergestellte Hohlzylinder verstreckt werden.

Bei einer alternativen Ausführungsform des Verfahrens wird zunächst eine Folie hergestellt und verstreckt (Schritte a) bis d)) und erst danach zu einem Hohlzylinder aufgerollt. Dabei kann das Aufrollen entweder parallel oder senkrecht zur Verstreckungsrichtung erfolgen, wobei Hohlzylinder mit einer erhöhten Reißfestigkeit in Längsrichtung bzw. in Umfangsrichtung erhalten werden. Je nach Anwendungsgebiet kann die eine oder die andere Variante bevorzugt sein.

Ein Aufrollen von Folien senkrecht zur Verstreckungsrichtung ist insbesondere bei faserverstärkten Folien vorteilhaft, da in diesem Fall die Fasern zumindest partiell entlang der Umfangsrichtung des Hohlzylinders orientiert sind. Bei einer Verwendung als Nervenleitschiene, die an ihren Enden häufig chirurgisch vernäht wird, kann eine deratige Faserorientierung einem Ausreißen des Nähfadens entgegenwirken.

Das erfindungsgemäße Verfahren eignet sich insbesondere zur Herstellung der oben beschriebenen erfindungsgemäßen Formkörper. Weitere Vorteile des Herstellungsverfahrens, ergeben sich somit aus der Beschreibung der erfindungsgemäßen Formkörper.

Die Erfindung betrifft des Weiteren die Verwendung der beschriebenen Formkörper für den Einsatz im human- und veterinärmedizinischen Bereich und für die Herstellung von Implantaten.

Eine erfindungsgemäße Verwendung betrifft zum einen die Herstellung von Wundauflagen aus den vorstehend beschriebenen Formkörpern. Diese können bei der Behandlung von Wunden oder von inneren oder äußeren Blutungen, z.B. bei Operationen, eingesetzt werden. Die Resorption des Formkörpers erfolgt dabei nach einer individuell einstellbaren Zeit, bevorzugt durch die Wahl der Herstellungsbedingungen.

Es hat sich gezeigt, dass die erfindungsgemäßen Formkörper hervorragend für die Besiedlung mit Säugetierzellen, d.h. mit menschlichen oder tierischen Zellen, geeignet sind. Dabei wird ein Formkörper mit einem geeigneten Nährmedium behandelt und anschließend die Zellen, z.B. Fibroblasten oder Chondrozyten, darauf ausgesät. Aufgrund der Stabilität des Materials können die Zellen *in vitro* mehrere Wochen wachsen und proliferieren.

Die Erfindung betrifft weiterhin Implantate, insbesondere Gewebeimplantate, die einen erfindungsgemäßen Formkörper und auf diesen aufgebrachte oder kultivierte Zellen, wie oben beschrieben, umfassen.

Die erfindungsgemäßen Implantate werden für die Behandlung von Gewebedefekten, beispielsweise Haut- oder Knorpeldefekten, verwendet, wobei die ausgesäten Zellen z.B. zuvor dem Patienten entnommen werden können. Während der Wachstumsphase der Zellen vermittelt der Formkörper dem sich bildenden Gewebe Schutz vor mechanischer Beanspruchung, und die Ausbildung der zelleigenen extrazellulären Matrix wird ermöglicht. Sowohl die hohe mechanische Festigkeit als auch die einstellbare Resorptionszeit der erfindungsgemäßen Formkörper erweisen sich dabei als besonderer Vorteil. Mit Hilfe langlebiger Materialien, die eine Resorptionszeit von mehr als vier Wochen aufweisen, können auch großflächige Defekte oder Defekte in Gewebetypen mit langsamem Zellwachstum behandelt werden.

Die Erfindung betrifft schließlich eine Nervenleitschiene, umfassend einen erfindungsgemäßen Formkörper in Form einer Hohlzylinders. Besondere Vorteile und Ausführungsformen derartiger Nervenleitschienen wurden bereits oben ausführlich beschrieben.

Diese und weitere Vorteile der Erfindung werden anhand der nachfolgenden, Beispiele unter Bezugnahme auf die Figuren näher erläutert. Es zeigen im Einzelnen:
- Figur 1:: Zug/Dehnungsdiagramm von erfindungsgemäßen Formkörpern in Form von Folien mit unterschiedlichem Vernetzungsgrad bei Verstreckung nach einer Lagerzeit von drei Tagen;
- Figur 2:: Zug/Dehnungsdiagramm von erfindungsgemäßen Formkörpern in Form von Folien mit unterschiedlichem Vernetzungsgrad bei Verstreckung nach einer Lagerzeit von sieben Tagen;
- Figur 3:: Zug/Dehnungsdiagramm von erfindungsgemäßen Formkörpern in Form von Folien mit unterschiedlichem Vernetzungsgrad bei Verstreckung nach einer Lagerzeit von 28 Tagen;
- Figur 4:: Zug/Dehnungsdiagramm von erfindungsgemäßen Formkörpern in Form von Folien mit unterschiedlichem Anteil an Weichmacher bei Verstreckung nach einer Lagerzeit von drei Tagen;
- Figur 5:: Zug/Dehnungsdiagramm von erfindungsgemäßen Formkörpern in Form von Folien mit unterschiedlichem Anteil an Weichmacher bei Verstreckung nach einer Lagerzeit von sieben Tagen;
- Figur 6:: Zug/Dehnungsdiagramm von erfindungsgemäßen Formkörpern in Form von Folien mit unterschiedlichem Anteil an Weichmacher bei Verstreckung nach einer Lagerzeit von 28 Tagen;
- Figur 7:: fotografische Darstellung erfindungsgemäßer Hohlzylinder; und
- Figur 8:: lichtmikroskopische Aufnahme eines erfindungsgemäßen Hohlzylinders im Querschnitt.

### Beispiel 1: Herstellung und Eigenschaften von verstreckten und unverstreckten Folien mit unterschiedlichem Vernetzungsgrad

Für dieses Beispiel wurden verschiedene Folien auf Basis eines Materials hergestellt, das jeweils konstante Anteile von ca. 71 Gew.% Gelatine und ca. 29 Gew.% Weichmacher enthielt. Die unterschiedlichen Mengen an Vernetzungsmittel lagen zwischen 1.000 und 4.000 ppm (jeweils bezogen auf die Menge an Gelatine).

Hierfür wurden pro Ansatz jeweils 20 g Schweineschwartengelatine mit einer Bloomstärke von 300 g in einer Mischung aus 72 g Wasser und 8 g Glycerin als Weichmacher bei 60 °C gelöst. Nach dem Entgasen der Lösungen durch Ultraschall wurde jeweils die in Tabelle 1 bezeichnete Menge einer wässrigen Formaldehydlösung (2,0 Gew.%ig, Raumtemperatur) zugegeben, die Mischung homogenisiert und bei ca. 60 °C in einer Dicke von 1 mm auf eine Polyethylenunterlage gerakelt.

**Tabelle 1**

| | | | | |
|---|---|---|---|---|
| Ansatz | 1-1 | 1-2 | 1-3 | 1-4 |
| Formaldehydlösung | 1 g | 2 g | 3 g | 4 g |
| Formaldehydgehalt bezogen auf Gelatine | 1.000 ppm | 2.000 ppm | 3.000 ppm | 4.000 ppm |

Nach dem Trocknen bei 25 °C und einer relativen Luftfeuchtigkeit von 30% während etwa zwei Tagen wurden die hergestellten Folien von der PE-Unterlage abgezogen. Die Dicke der Folien betrug ca. 220 µm.

Vor dem Verstrecken wurden verschiedene, gemäß den obigen Ansätzen 1-1 bis 1-4 hergestellte Folien für drei, sieben bzw. 28 Tage bei einer Temperatur von 23 °C und einer relativen Luftfeuchtigkeit von 45% gelagert. Entsprechende Vergleichsfolien, die nicht verstreckt wurden, wurden jeweils auf gleiche Weise behandelt.

Für das Verstrecken wurden die Folien unter Einwirkung von heißem Wasserdampf erweicht, in diesem thermoplastischen Zustand in einer Richtung bis zur Verstreckungsgrenze gelängt und über Nacht bei einer bei einer Temperatur von 23 °C und einer relativen Luftfeuchtigkeit von 45% fixiert. Die Verstrekkungsverhältnisse lagen dabei in einem Bereich von ca. 2 bis 4.

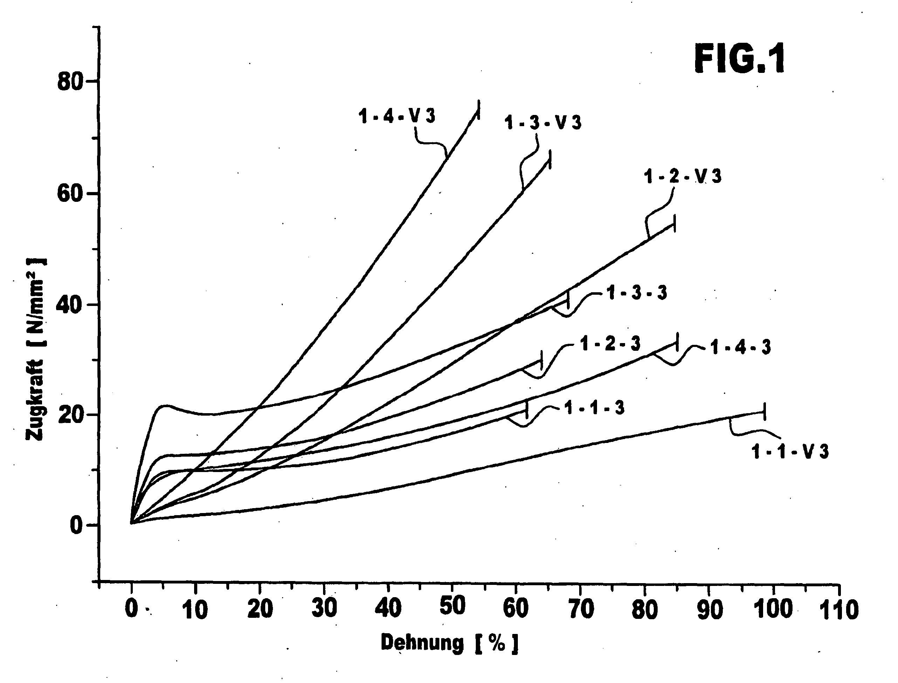

Anschließend wurden die Zug/Dehnungsdiagramme der verstreckten Folien (in Verstreckungsrichtung) sowie der entsprechenden unverstreckten Folien aufgenommen. Diese sind in den Figuren 1 bis 3 dargestellt.

Bei der Beschriftung der einzelnen Kurven in den Schaubildern stehen jeweils die ersten beiden Ziffern für den Ansatz, nach dem die Folie hergestellt wurde, während die dritte Ziffer für die Lagerzeit der Folie vor dem Verstrecken steht (drei, sieben bzw. 28 Tage). Verstreckte Folien sind durch den Buchstaben V vor der letzten Ziffer gekennzeichnet.

Figur 1 zeigt das Zug/Dehnungsdiagramm der nach drei Tagen verstreckten Folien sowie der unverstreckten Vergleichsfolien, die drei Tage bei denselben Bedingungen gelagert wurden. Ein Vergleich der Kurven untereinander zeigt zunächst, dass die Reißfestigkeit der erfindungsgemäß verstreckten Folien mit der Erhöhung des Gehaltes an Vernetzungsmittel deutlich zunimmt.

Auch die Auswirkungen der Verstreckung sind vom Gehalt an Vernetzungsmittel abhängig. Bei dem relativ geringen Formaldehydgehalt von 1.000 ppm bleibt die Reißfestigkeit der verstreckten Folie 1-1-V3 gegenüber der unverstreckten Folie 1-1-3 weitgehend konstant, während sich die Reißdehnung von ca. 60% auf fast 100% deutlich erhöht. Bei Formaldehydkonzentrationen von 2.000 ppm und mehr führt das Verstrecken zu Folien mit einer deutlich erhöhten Reißfestigkeit, im Falle von 4.000 ppm Formaldehyd sogar auf mehr als das Doppelte (Folie 1-4-V3 gegenüber Folie 1-4-3).

Diese Ergebnisse zeigen, dass durch das Verstrecken von Folien auf Basis von vernetzter Gelatine deren mechanische Eigenschaften auf vielfältige Weise verbessert werden können. Je nach Vernetzungsgrad ergibt sich ein positiver Einfluss auf die Reißdehnung, die Reißfestigkeit oder auch auf beide Parameter zugleich (z.B. Folie 1-2-V3 gegenüber Folie 1-2-3).

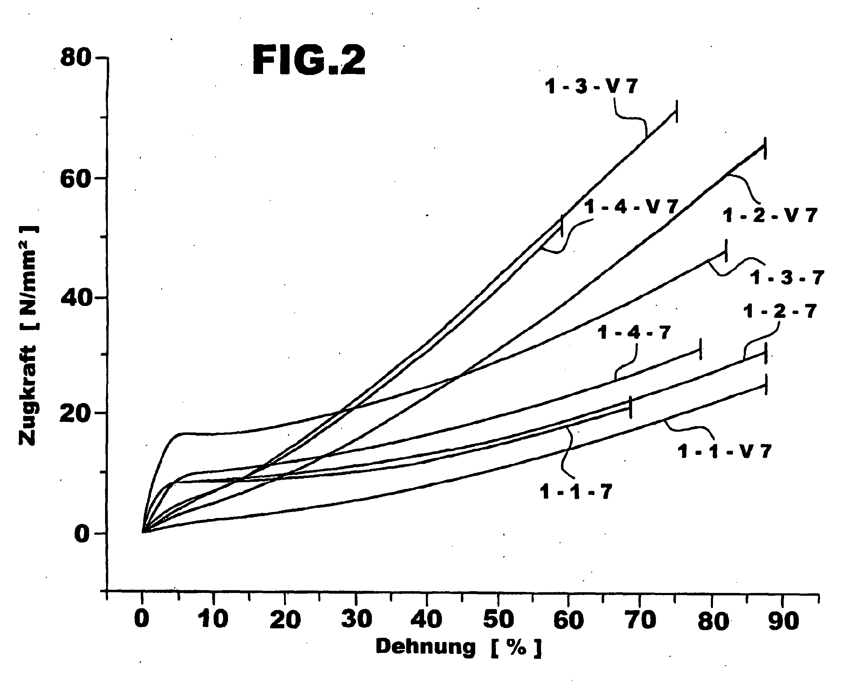

Figur 2 zeigt das Zug/Dehnungsdiagramm der nach sieben Tagen verstreckten Folien sowie der entsprechenden Vergleichsfolien. Die durch das Verstrecken erzielte höhere Reißfestigkeit der Folien ist auch hier deutlich erkennbar.

Ein Vergleich mit der Figur 1 zeigt zudem, dass durch die längere Lagerzeit vor dem Verstrecken bereits bei niedrigeren Vernetzergehalten höhere Reißfestigkeiten der erfindungsgemäßen Folien erzielt werden können (z.B. Folie 1-2-V7 gegenüber Folie 1-2-V3). Ursache hierfür ist vermutlich ein Weiterlaufen der Vernetzungsreaktion während der Lagerzeit.

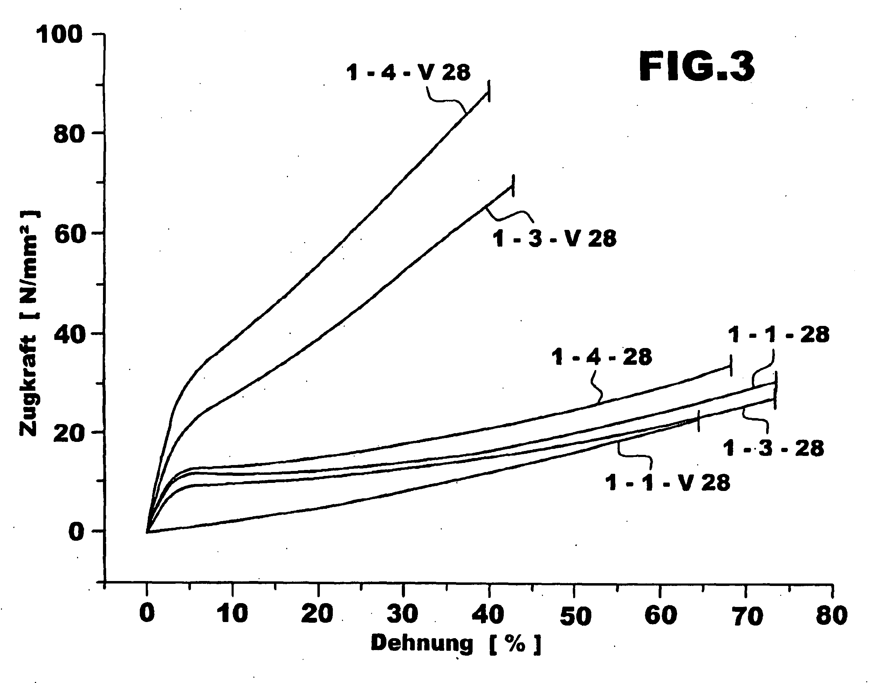

Figur 3 zeigt schließlich die mechanischen Eigenschaften der nach 28 Tagen verstreckten Folien sowie der entsprechenden Vergleichsfolien. Die Zug/Dehnungsdiagramme wurden hier nur für Folien gemäß den Ansätzen 1-1, 1-3 und 1-4 aufgenommen.

Während die Kurvenverläufe der unverstreckten Folien nach einer Lagerzeit von 28 Tagen nahezu identisch sind, sind die Eigenschaften der verstreckten Folien in hohem Maße vom Vernetzungsmittelgehalt abhängig. Bei einem geringen Gehalt von 1.000 ppm, hat das Verstrecken kaum einen Einfluss, bei 3.000 und 4.000 ppm steigt hingegen die Reißfestigkeit gegenüber den unverstreckten Folien dramatisch an. Die maximale Reißfestigkeit von fast 90 N/mm², die bei der Folie 1-4-V28 erreicht wird, ist durch die lange Lagerzeit nochmals höher als bei den nach drei oder sieben Tagen verstreckten Folien.

Bei sämtlichen dargestellten Zug/Dehnungsdiagrammen ist zu berücksichtigen, dass die jeweiligen Kurvenverläufe bei Herstellung von Folien im Labormaßstab nicht exakt reproduzierbar sind. Charakteristisch ist jedoch das Verhältnis der Kurven verschiedener Folien zueinander.

### Beispiel 2: Herstellung und Eigenschaften von verstreckten und unverstreckten Folien mit unterschiedlichen Anteilen an Weichmacher

Dieses Beispiel betrifft Folien auf Basis von vernetzter Gelatine mit einem konstanten Gehalt an Vernetzungsmittel von 2.000 ppm (bezogen auf die Menge an Gelatine). Das Material für die Folien umfasste dabei neben Gelatine unterschiedlich Anteile an Weichmacher zwischen ca. 17 Gew.% und ca. 33 Gew.%.

Zur Herstellung der Folien wurden jeweils 20 g Schweineschwartengelatine (Bloomstärke 300 g) in vier verschiedenen Ansätzen in einer Mischung aus Wasser und Glycerin als Weichmacher, jeweils entsprechend den Mengenangaben in der Tabelle 2, bei 60 °C gelöst. Nach dem Entgasen der Lösung durch Ultraschall wurden jeweils 2 g einer wässrigen Formaldehydlösung (2,0 gew.%ig, Raumtemperatur) zugegeben, die Mischung homogenisiert und bei ca. 60 °C in einer Dicke von 1 mm auf eine Polyethylenunterlage gerakelt.

**Tabelle 2**

| | | | | |
|---|---|---|---|---|
| Ansatz | 2-1 | 2-2 | 2-3 | 2-4 |
| Wasser | 76 g | 74 g | 72 g | 70 g |
| Glycerin | 4 g | 6 g | 8 g | 10 g |
| Anteil an Glycerin in dem Material | 16,7 Gew.% | 23,1 Gew.% | 28,6 Gew.% | 33,3 Gew.% |

Das Trocknen, Lagern und Verstrecken der Folien erfolgte auch hier wie in Beispiel 1 beschrieben.

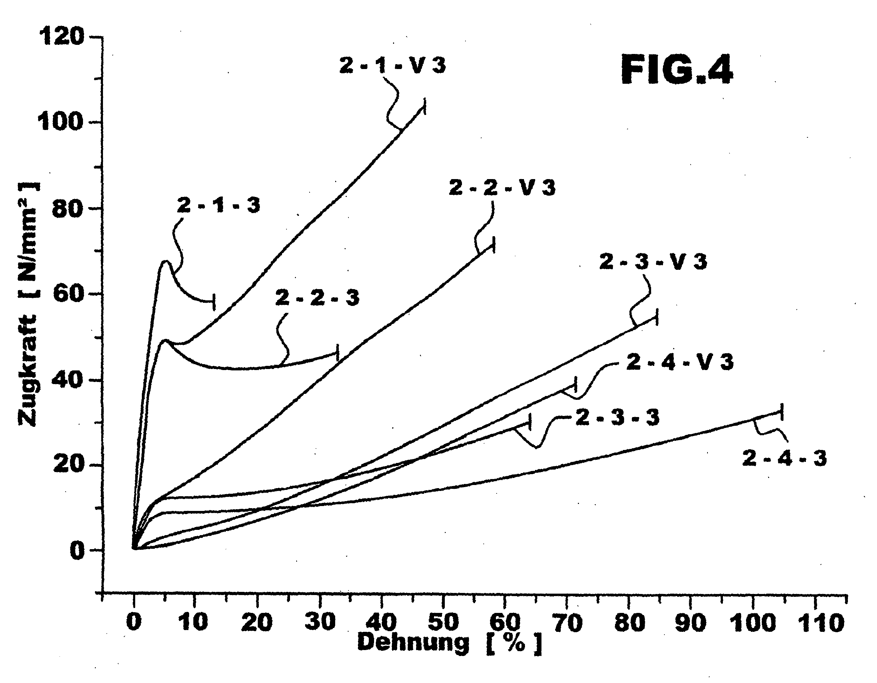

Die Zug/Dehnungsdiagramme der verstreckten und unverstreckten Folien sind in den Figuren 4 bis 6 dargestellt. Die Bezeichnungen der einzelnen Kurven sind analog zu Beispiel 1.

Figur 4 zeigt die Zug/Dehnungsdiagramme der erfindungsgemäßen Folien, die nach einer Lagerzeit von drei Tagen verstreckt wurden, sowie der entsprechenden unverstreckten Vergleichsfolien. Zunächst fällt auf, dass bei sämtlichen eingesetzten Anteilen an Weichmacher die Reißfestigkeit der erfindungsgemäßen Folien durch das Verstrecken deutlich erhöht wird. Besonders auffallend ist dieser Effekt bei den Folien der Ansätze 2-1 und 2-2 mit geringerem Weichmacheranteil, die ohne Verstreckung ein völlig unbefriedigendes Zug/Dehnungsverhalten aufweisen. Die verstreckten Folien weisen demgegenüber sehr gute mechanische Eigenschaften mit hohen Reißfestigkeiten auf (ca. 100 N/mm² bei der Folie 2-1-V3).

Zu beachten ist weiterhin, dass durch das erfindungsgemäße Verstrecken der Folien nicht nur die Reißfestigkeit, sondern mit Ausnahme des Ansatzes 2-4 auch die Reißdehnung der Folien signifikant verbessert wird. Dies ist erstaunlich, wenn man berücksichtigt, dass die Folien bereits beim Verstrecken eine Dehnung von ca. 100 bis 300% erfahren haben.

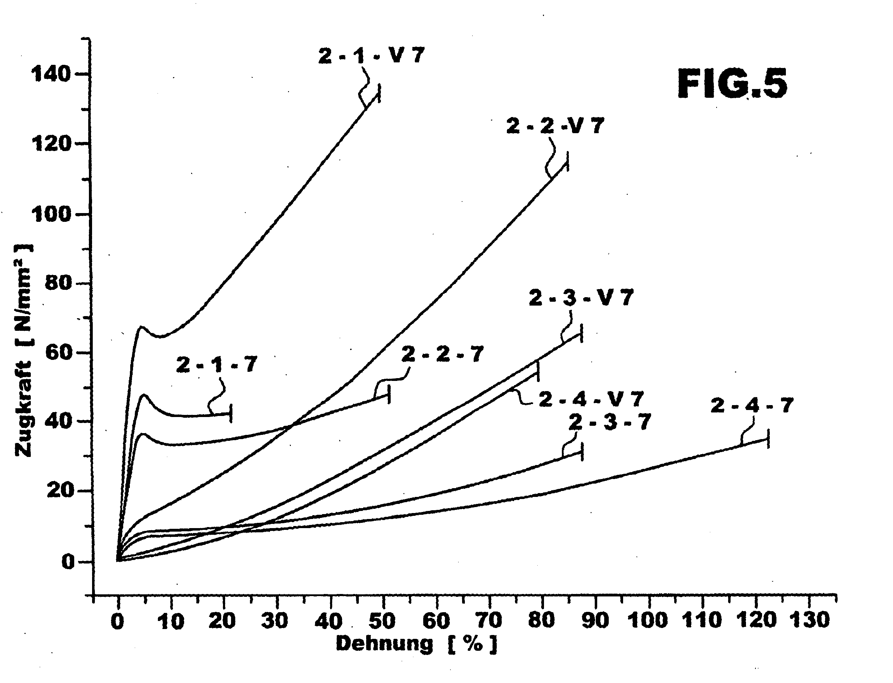

Die Zug/Dehnungsdiagramme der nach sieben Tagen verstreckten Folien in der Figur 5 zeigen qualitativ dieselben Ergebnisse wie beim Verstrecken nach drei Tagen. Bei allen Ansätzen ist die Reißfestigkeit der erfindungsgemäß verstreckten Folien durch die längere Lagerzeit zum Teil deutlich erhöht, was in erster Linie auf das oben beschriebene Weiterlaufen der Vernetzungsreaktion zurückzuführen sein dürfte. Auch auf die Reißdehnungen hat die längere Lagerung einen positiven Einfluss.

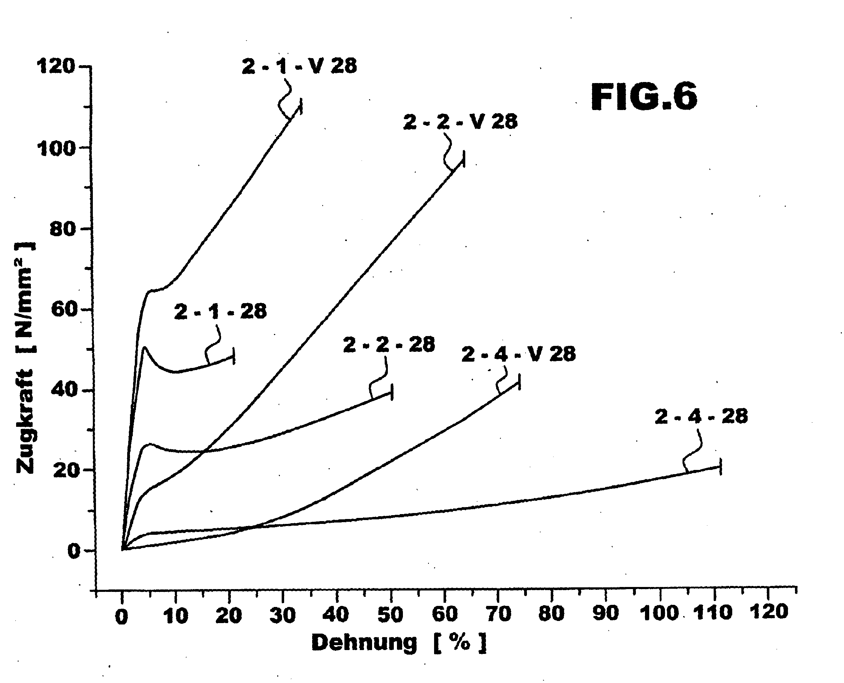

Figur 6 zeigt schließlich die Zug/Dehnungsdiagramme der Folien bei einer Lagerzeit von 28 Tagen, wobei hier nur die verstreckten und die unverstreckten Folien der Ansätze 2-1, 2-2 und 2-4 vermessen wurden. Im Vergleich zur Figur 5 weisen die Kurven einen sehr ähnlichen Verlauf auf, wobei die Reißfestigkeiten der verstreckten Folien sogar wieder etwas geringer sind als bei der siebentägigen Lagerung. Dies deutet darauf hin, dass es ein Optimum für die Lagerzeit gibt, welches von der Vernetzungsmittelkonzentration und dem Anteil an Weichmacher abhängig sein dürfte.

### Beispiel 3: Herstellung zweifach vernetzter, verstreckter Folien

Dieses Beispiel betrifft die Herstellung erfindungsgemäßer Folien mit einem zweiten Vernetzungsschritt nach dem Verstrecken, wodurch die physiologischen Abbauzeiten der Folien deutlich verlängert werden.

Ausgangspunkt hierfür waren die verstreckten Folien der Beispiele 1 und 2. Diese wurden, nachdem sie verstreckt und über Nacht fixiert worden waren, in einem Exsikkator für zwei Stunden dem Gleichgewichtsdampfdruck einer 17 gew.%igen wässrigen Formaldehydlösung bei Raumtemperatur ausgesetzt.

Anschließend wurde das Abbauverhalten dieser zweifach vernetzten Folien im Unterschied zu den einfach vernetzten Ausgangsfolien untersucht. Hierzu wurden 2 x 3 cm große Folienstücke in jeweils 500 ml PBS-Puffer (pH 7,2) eingelegt und photometrisch die Konzentration der im Puffer gelösten Gelatine bei einer Wellenlänge von 214 nm gemessen. Während die einfach vernetzten Folien bereits nach 15 Minuten vollständig aufgelöst waren, war an den zweifach vernetzten Folien nach einer Stunde noch keine Veränderung festzustellen.

Die vorteilhaften mechanischen Eigenschaften der verstreckten Folien blieben durch den zweiten Vernetzungsschritt im Wesentlichen unbeeinflusst.

### Beispiel 4: Herstellung enzymatisch vernetzter Folien auf Basis von Gelatine

Dieses Beispiel betrifft die Herstellung einer Folie auf Basis von Gelatine, wobei die Vernetzung enzymatsich mit Transglutaminase durchgeführt wurde.

Hierfür wurden 20 g Schweineschwartengelatine (Bloomstärke 300 g) in einer Mischung aus 72 g Wasser und 8 g Glycerin, was einem Anteil an Weichmacher von ca. 29 Gew.% entspricht, bei 60°C gelöst. Nach dem Entgasen der Lösung durch Ultraschall wurden 4 g einer wässrigen Transglutaminase-Lösung mit einer spezifischen Aktivität von 30 U/g zugegeben, die Mischung homogenisiert und in einer Dicke von 1 mm auf eine auf 45 °C temperierte Polyethylenunterlage gerakelt.

Nach 30 Minuten wurde die Folie von der PE-Unterlage abgezogen, 2 Stunden bei einer Temperatur von 50 °C und einer relativen Luftfeuchtigkeit von 90% gehalten und anschließend für etwa zwei Tage bei einer Temperatur von 25 °C und einer relativen Luftfeuchtigkeit von 30% getrocknet.

Die mit Transglutaminase vernetzt Folie wies eine Reißfestigkeit von ca. 9 N/mm² bei einer Reißdehnung von ca. 300% auf.

Eine Verstreckung der auf diese Weise hergestellten Folie und ggf. eine zweite Vernetzung mit Formaldehyd in der Gasphase können auf gleiche Weise durchgeführt werden, wie dies in den Beispielen 1 bzw. 3 beschrieben ist.

### Beispiel 5: Herstellung von verstreckten Hohlzylindern auf Basis von Gelatine

Durch das erfindungsgemäße Verstrecken von Hohlzylindern auf Basis von Gelatine konnten sehr dünne Röhrchen mit einem Innendurchmesser im Bereich von 800 bis 1.200 µm hergestellt werden.

Als Ausgangsmaterial diente eine Lösung von Schweineschwartengelatine (Bloomstärke 300 g), die entsprechend der in den Beispielen 1 und 2 beschriebenen Vorgehensweise durch Lösen von 100 g Gelatine in einer Mischung aus 260 g Wasser und 40 g Glycerin als Weichmacher hergestellt wurde. Dies entspricht einem Anteil an Weichmacher von ca. 29 Gew.%.

Nach Zugabe von 4 g einer wässrigen, 2,0 Gew.%igen Formaldehydlösung (800 ppm Vernetzer bezogen auf die Gelatine) wurde die Lösung homogenisiert, nochmals entgast und die Oberfläche von Schaum befreit. Eine Reihe von Edelstahlpins mit einem Durchmesser von 2 mm, die zuvor mit einem Trennwachs eingesprüht worden waren, wurden in einer Länge von ca. 3 cm kurz in die Lösung eingetaucht. Nach dem Herausziehen der Pins aus der Lösung wurden diese senkrecht gehalten, so dass die anhaftende Lösung eine möglichst gleichmäßige Schicht bildete.

Nach etwa eintägigem Trocknen bei 25 °C und einer relativen Luftfeuchtigkeit von 30% konnten die gebildeten Gelatineröhrchen von den Edelstahlpins abgezogen werden. Diese wurden dann weitere fünf Tage bei 23 °C und einer relativen Luftfeuchtigkeit von 45% gelagert.

Zum Verstrecken wurden die Röhrchen an beiden Enden eingespannt und unter Einwirkung von heißem Wasserdampf erweicht. In diesem thermoplastischen Zustand wurden sie mit einem Verstreckungsverhältnis von ca. 1,4 gelängt, in diesem Zustand fixiert und über Nacht bei 23 °C und einer relativen Luftfeuchtigkeit von 45% getrocknet.

Um die physiologische Abbauzeit der Gelatineröhrchen zu verlängern, wurden diese, entsprechend den in Beispiel 3 beschriebenen Folien, einem zweiten Vernetzungsschritt unterzogen. Hierzu wurden die Röhrchen in einem Exsikkator für 17 Stunden dem Gleichgewichtsdampfdruck einer 17 gew.%igen wässrigen Formaldehydlösung bei Raumtemperatur ausgesetzt. Dabei wurden beide Enden der Röhrchen verschlossen, so dass die Vernetzung im Wesentlichen nur von der Außenseite her erfolgte.

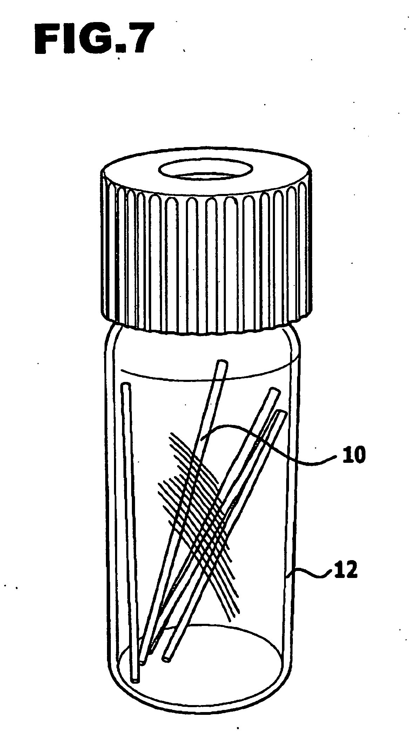

In der Figur 7 sind einige der auf diese Weise hergestellten Gelatineröhrchen 10 mit einer Länge von ca. 3 cm in einem Glasgefäß 12 dargestellt.

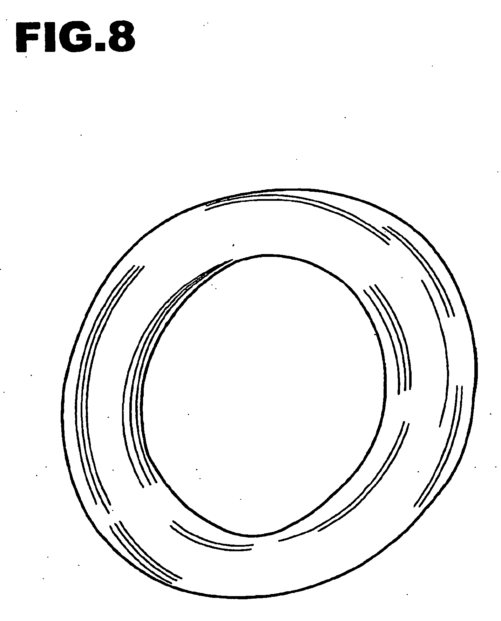

Figur 8 zeigt eine lichtmikroskopische Aufnahme des Querschnitts durch eines der Röhrchen. Das abgebildete Röhrchen weist einen Innendurchmesser von ca. 1.100 µm und eine Wandstärke von ca. 200 µm auf: Sowohl die Querschnittsform als auch die Wandstärke des Röhrchens sind äußerst regelmäßig.

Die in diesem Beispiel hergestellten Gelatineröhrchen eignen sich aufgrund ihrer Abmessungen und aufgrund ihrer langen Abbauzeiten besonders gut zur Verwendung als Nervenleitschienen. Auch die stärkere Vernetzung der Röhrchen von der Außenseite her ist für diese Anwendung vorteilhaft, da hierdurch das Röhrchen im Zuge des Wachstums der Nervenzelle von innen heraus abgebaut werden kann.

Durch eine Erhöhung des Verstreckungsverhältnisses können auch erfindungsgemäße Hohlzylinder mit einem noch kleineren Innendurchmesser hergestellt werden, die für andere Anwendungen vorteilhaft sein können. Insbesondere ist es unter Anwendung des erfindungsgemäßen Verfahrens möglich, äußerst dünne Röhrchen mit einem Durchmesser im Bereich von 150 µm herzustellen. Ein solcher Wert kann ohne ein Verstrecken der Röhrchen nicht erreicht werden.

## Patentansprüche

1. Formkörper auf Basis eines vernetzten, Gelatine enthaltenden Materials, wobei der Formkörper verstreckt ist, so dass die Gelatinemoleküle zumindest teilweise in einer Vorzugsrichtung orientiert sind, und wobei das Material einen Weichmacher umfasst.

2. Formkörper nach Anspruch 1, wobei das Material zu überwiegenden Anteilen aus Gelatine gebildet ist.

3. Formkörper nach Anspruch 1 oder 2, wobei der Weichmacher ausgewählt ist aus Glycerin, Oligoglycerinen, Oligoglykolen und Sorbit.

4. Formkörper nach einem der vorangehenden Ansprüche, wobei der Formkörper monoaxial verstreckt ist.

5. Formkörper nach einem der vorangehenden Ansprüche, wobei der Formkörper einen Verstärkungsstoff umfasst.

6. Formkörper nach Anspruch 5, wobei der Verstärkungsstoff ausgewählt ist aus partikulären und/oder molekularen Verstärkungsstoffen.

7. Formkörper nach Anspruch 6, wobei der partikuläre Verstärkungsstoff Verstärkungsfasern umfasst.

8. Formkörper nach einem der vorangehenden Ansprüche, wobei der Formkörper eine Folie ist.

9. Formkörper nach einem der Ansprüche 1 bis 7, wobei der Formkörper ein Hohlzylinder ist.

10. Formkörper nach Anspruch 9, wobei der Hohlzylinder in Längsrichtung verstreckt ist.

11. Formkörper nach Anspruch 9, wobei der Hohlzylinder in Umfangsrichtung verstreckt ist.

12. Verfahren zur Herstellung eines verstreckten Formkörpers auf Basis eines vernetzten, Gelatine enthaltenden Materials, umfassend folgende Schritte:
a) Herstellen einer wässrigen Lösung eines Gelatine enthaltenden Materials;
b) partielles Vernetzen des gelösten, Gelatine enthaltenden Materials;
c) Herstellen eines Formkörpers ausgehend von der das partiell vernetzte Material enthaltenden Lösung; und
d) Verstrecken des Formkörpers.

13. Verfahren nach Anspruch 12, wobei das Material in Schritt a) zu überwiegenden Anteilen aus Gelatine gebildet ist.

14. Verfahren nach Anspruch 12 oder 13, wobei das Material in Schritt a) einen Weichmacher umfasst.

15. Verfahren nach einem der Ansprüche 12 bis 14, wobei der Formkörper zwischen den Schritten c) und d) zumindest teilweise getrocknet wird.

16. Verfahren nach einem der Ansprüche 12 bis 15, wobei der Formkörper unmittelbar vor Schritt d) durch eine Erhöhung der Temperatur und/oder des Wassergehalts in einen thermoplastischen Zustand überführt wird.

17. Verfahren nach einem der Ansprüche 12 bis 16, wobei Schritt d) mit einem Verstreckungsverhältnis von 1,4 bis 8 durchgeführt wird.

18. Verfahren nach einem der Ansprüche 12 bis 17, wobei Schritt d) drei bis sieben Tage nach Schritt c) durchgeführt wird.

19. Verfahren nach einem der Ansprüche 12 bis 18, ferner umfassend:
e) Vernetzen des in dem verstreckten Formkörper enthaltenen Materials.

20. Verfahren nach Anspruch 19, wobei die Vernetzung in Schritt e) durch das Einwirken eines Vernetzungsmittels in der Gasphase durchgeführt wird.

21. Verfahren nach einem der Ansprüche 12 bis 20, wobei der Formkörper eine Folie ist.

22. Verfahren nach einem der Ansprüche 12 bis 20, wobei der Formkörper ein Hohlzylinder ist.

23. Verwendung eines Formkörpers nach einem der Ansprüche 1 bis 11 für die Herstellung eines resorbierbaren Materials zur Abdeckung von Wunden oder von inneren oder äußeren Blutungen im human- oder veterinärmedizinischen Bereich.

24. Verwendung eines Formkörpers nach einem der Ansprüche 1 bis 11 als Träger für die Kultivierung von Säugetierzellen *in vitro.*

25. Implantat, umfassend einen Formkörper nach einem der Ansprüche 1 bis 11 sowie Säugetierzellen, die auf dem Formkörper aufgebracht oder kultiviert sind.

## Claims

1. Shaped body based on a cross-linked, gelatinous material, the shaped body being stretched so that the gelatin molecules are oriented at least in part in a preferred direction, and the material comprising a plasticizer.

2. Shaped body according to claim 1, the material being formed in preponderant proportions from gelatin.

3. Shaped body according to claim 1 or 2, the plasticizer being selected from glycerin, oligoglycerins, oligoglycols and sorbite.

4. Shaped body according to any of the preceding claims, the shaped body being stretched monoaxially.

5. Shaped body according to any of the preceding claims, the shaped body comprising a reinforcing material.

6. Shaped body according to claim 5, the reinforcing material being selected from particulate and/or molecular reinforcing materials.

7. Shaped body according to claim 6, the particulate reinforcing material comprising reinforcing fibres.

8. Shaped body according to any of the preceding claims, the shaped body being a film.

9. Shaped body according to any of claims 1 to 7, the shaped body being a hollow cylinder.

10. Shaped body according to claim 9, the hollow cylinder being stretched in the longitudinal direction.

11. Shaped body according to claim 9, the hollow cylinder being stretched in the circumferential direction.

12. Method for producing a stretched shaped body based on a cross-linked, gelatinous material, comprising the following steps:
a) preparing an aqueous solution of a gelatinous material;
b) partially cross-linking the dissolved, gelatinous material;
c) producing a shaped body starting from the solution containing the partially cross-linked material; and
d) stretching the shaped body.

13. Method according to claim 12, the material in step a) being formed in preponderant proportions from gelatin.

14. Method according to claim 12 or 13, the material in step a) comprising a plasticizer.

15. Method according to any of claims 12 to 14, the shaped body being at least partially dried between steps c) and d).

16. Method according to any of claims 12 to 15, the shaped body being brought into a thermoplastic state directly before step d) by raising the temperature and/or the water content.

17. Method according to any of claims 12 to 16, step d) being carried out with a stretch ratio of 1.4 to 8.

18. Method according to any of claims 12 to 17, step d) being carried out three to seven days after step c).

19. Method according to any of claims 12 to 18, further comprising:
e) cross-linking the material contained in the stretched shaped body.

20. Method according to claim 19, the cross-linking in step e) being carried out by the action of a cross-linking agent in the gas phase.

21. Method according to any of claims 12 to 20, the shaped body being a film.

22. Method according to any of claims 12 to 20, the shaped body being a hollow cylinder.

23. Use of a shaped body according to any of claims 1 to 11 for producing a resorbable material for covering wounds or internal or external bleeding in the fields of human or veterinary medicine.

24. Use of a shaped body according to any of claims 1 to 11 as a carrier for cultivating mammalian cells *in vitro.*

25. Implant, comprising a shaped body according to any of claims 1 to 11 and mammalian cells which are applied to or cultivated on the shaped body.

## Revendications

1. Corps de forme façonné à base d'un matériau réticulé renfermant de la gélatine, le corps de forme façonné étant étiré, de sorte que les molécules de gélatine sont orientées au moins partiellement dans une direction préférentielle, et dans lequel ledit matériau comprend un plastifiant.

2. Corps de forme façonné selon la revendication 1, dans lequel le matériau est constitué selon des proportions prépondérantes, de gélatine.

3. Corps de forme façonné selon la revendication 1 ou 2, dans lequel le plastifiant est choisi parmi la glycérine, les oligoglycérines, les oligoglycols et le sorbitol.

4. Corps de forme façonné selon l'une des revendications précédentes, le corps de forme façonné étant étiré de manière monoaxiale.

5. Corps de forme façonné selon l'une des revendications précédentes, le corps de forme façonné renfermant une substance de renfort.

6. Corps de forme façonné selon la revendication 5, dans lequel la substance de renfort est choisie parmi des substances de renfort particulaires et/ou moléculaires.

7. Corps de forme façonné selon la revendication 6, dans lequel la substance de renfort particulaire comprend des fibres de renfort.

8. Corps de forme façonné selon l'une des revendications précédentes, le corps de forme façonné étant une feuille.

9. Corps de forme façonné selon l'une des revendications 1 à 7, le corps de forme façonné étant un cylindre creux.

10. Corps de forme façonné selon la revendication 9, dans lequel le cylindre creux est étiré dans la direction longitudinale.

11. Corps de forme façonné selon la revendication 9, dans lequel le cylindre creux est étiré dans la direction périphérique.

12. Procédé de fabrication d'un corps de forme façonné étiré, à base d'un matériau réticulé renfermant de la gélatine, englobant les étapes suivantes :
a) fabrication d'une solution aqueuse d'un matériau renfermant de la gélatine ;
b) réticulation partielle du matériau en solution renfermant de la gélatine ;
c) fabrication d'un corps de forme façonné à partir de la solution renfermant le matériau partiellement réticulé ; et
d) étirage du corps de forme façonné.

13. Procédé selon la revendication 12, d'après lequel le matériau dans l'étape a) est constitué selon des proportions prépondérantes, de gélatine.

14. Procédé selon la revendication 12 ou 13, d'après lequel le matériau dans l'étape a) renferme un plastifiant.

15. Procédé selon l'une des revendications 12 à 14, d'après lequel le corps de forme façonné est séché, au moins en partie, entre les étapes c) et d).

16. Procédé selon l'une des revendications 12 à 15, d'après lequel le corps de forme façonné est transféré dans un état thermoplastique directement avant l'étape d), par élévation de la température et/ou de la teneur en eau.

17. Procédé selon l'une des revendications 12 à 16, d'après lequel l'étape d) est effectuée avec un rapport d'étirage de 1,4 à 8.

18. Procédé selon l'une des revendications 12 à 17, d'après lequel l'étape d) est effectuée trois à sept jours après l'étape c).

19. Procédé selon l'une des revendications 12 à 18, comprenant en outre :
e) la réticulation du matériau contenu dans le corps de forme façonné étiré.

20. Procédé selon la revendication 19, d'après lequel la réticulation dans l'étape e) est effectuée par l'action d'un agent de réticulation en phase gazeuse.

21. Procédé selon l'une des revendications 12 à 20, d'après lequel le corps de forme façonné est une feuille.

22. Procédé selon l'une des revendications 12 à 20, d'après lequel le corps de forme façonné est un cylindre creux.

23. Utilisation d'un corps de forme façonné selon l'une des revendications 1 à 11 pour la fabrication d'un matériau résorbable destiné à recouvrir des plaies ou des hémorragies internes ou externes dans le domaine de la médecine humaine ou vétérinaire.

24. Utilisation d'un corps de forme façonné selon l'une des revendications 1 à 11, en tant que support de culture de cellules de mammifères in vitro.

25. Implant, comprenant un corps de forme façonné selon l'une des revendications 1 à 11, ainsi que des cellules de mammifères, qui sont rapportées ou en culture sur le corps de forme façonné.
